# FASCICULE DE BREVET EUROPEEN

(11) **EP 2 139 900 B1**
(45) Date de publication et mention de la délivrance du brevet: **06.10.2010**
(21) Numéro de dépôt: 08788210.6
(22) Date de dépôt: 21.04.2008
(51) Int. Cl.: C07D 493/04, C07D 307/20

(54) **NOUVEAUX COMPOSES FONCTIONNELS A COEUR D'ISOSORBIDE OU D'ISOMERE D'ISOSORBIDE, PROCEDE DE FABRICATION ET APPLICATIONS DE CES COMPOSES**
NEUARTIGE FUNKTIONELLE VERBINDUNGEN MIT EINEM ISOSORBID- ODER EINEM ISOSORBIDISOMER-KERN SOWIE HERSTELLUNGSVERFAHREN FÜR DIESE VERBINDUNGEN UND IHRE ANWENDUNG
NOVEL FUNCTIONAL COMPOUNDS WITH AN ISOSORBIDE OR ISOSORBIDE ISOMER CORE, PRODUCTION PROCESS AND USES OF THESE COMPOUNDS

(30) Priorité: 27.04.2007 FR 0754772
(43) Date de publication de la demande: 06.01.2010
(73) Titulaire: ARKEMA FRANCE, 92700 Colombes (FR)
(72) Inventeur: GILLET, Jean-Philippe, F-69530 Brignais (FR)
(74) Mandataire: Sauvageot, Olivier Roger
(86) Numéro de dépôt international: PCT/FR2008/050711
(87) Numéro de publication internationale: WO 2008/145921

(56) Documents cités:
- DE-A1- 19 704 506
- GUO ET AL: "Molecular modeling analysis of the interaction of novel bis-cationic ligands with the lipid A moiety of lipopolysaccharide" BIOORGANIC & MEDICINAL CHEMISTRY LETTERS, OXFORD, GB, vol. 16, no. 3, 1 février 2006 (2006-02-01), pages 714-717, XP005222784 ISSN: 0960-894X cité dans la demande
- CAOUTHAR ET AL: "Synthesis and characterization of new polyamides derived from di(4-cyanophenyl)isosorbide" EUROPEAN POLYMER JOURNAL, PERGAMON PRESS LTD. OXFORD, GB, vol. 43, no. 1, 14 décembre 2006 (2006-12-14), pages 220-230, XP005803560 ISSN: 0014-3057
- CAOUTHAR, ASMAA A. ET AL: "Synthesis and characterization of new polyamides based on diphenylaminoisosorbide Synthesis and characterization of new polyamides based on diphenylaminoisosorbide" JOURNAL OF POLYMER SCIENCE, PART A: POLYMER CHEMISTRY , 43(24), 6480-6491 CODEN: JPACEC; ISSN: 0887-624X, 2005, XP002461541
- ZARIF L ET AL: "PERFLUOROALKYLATED MONOESTERS OF 1,4-D-SORBITAN, ISOSORBIDE AND ISOMANNIDE: NEW SURFACTANTS FOR BIOMEDICAL APPLICATIONS" JOURNAL OF FLUORINE CHEMISTRY, ELSEVIER, AMSTERDAM, NL, vol. 44, 1989, pages 73-85, XP001062724 ISSN: 0022-1139
- BAZIN H ET AL: "HYDROLYSIS OF CYANOETHYLATED CARBOHYDRATES: SYNTHESIS OF NEW CARBOXYLIC DERIVATIVES OF SUCROSE, D-GLUCOSEAND D-FRUCTOSE" JOURNAL OF CARBOHYDRATE CHEMISTRY, NEW YORK, NY, US, vol. 14, no. 8, 1995, pages 1187-1207, XP003017613 ISSN: 0732-8303
- "Chlorierung von Tetrahydrofuran und Reaktionen des 2,3-Dichlortetrahydrofurans" JUSTUS LIEBIGS ANNALEN DER CHEMIE, VERLAG CHEMIE GMBH. WEINHEIM, DE, vol. 596, 1955, pages 113-124, XP009093297 ISSN: 0075-4617

## Description

La présente invention porte sur de nouveaux composés fonctionnels qui comportent, comme motif de coeur, un motif isosorbide ou de l'un des deux isomères, optiques de l'isosorbide, à savoir l'isomannide ou l'isoidide. La présente invention porte également sur un procédé de préparation de ces nouveaux composés fonctionnels, ainsi que sur leurs applications.

L'isosorbide : 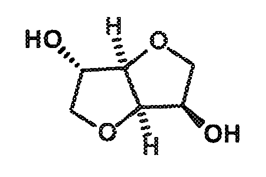 l'isomannide : 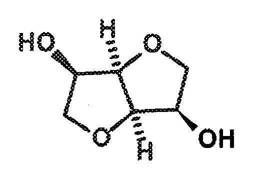 l'isoidide : 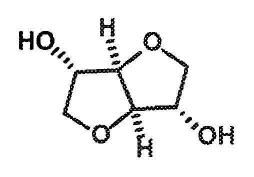 sont des substances naturelles obtenues principalement à partir des sucres issus de l'amidon de maïs. Ce dernier, par réaction enzymatique, donne le glucose, lequel est réduit en sorbitol, ce dernier conduisant à l'isosorbide après une double déshydratation :

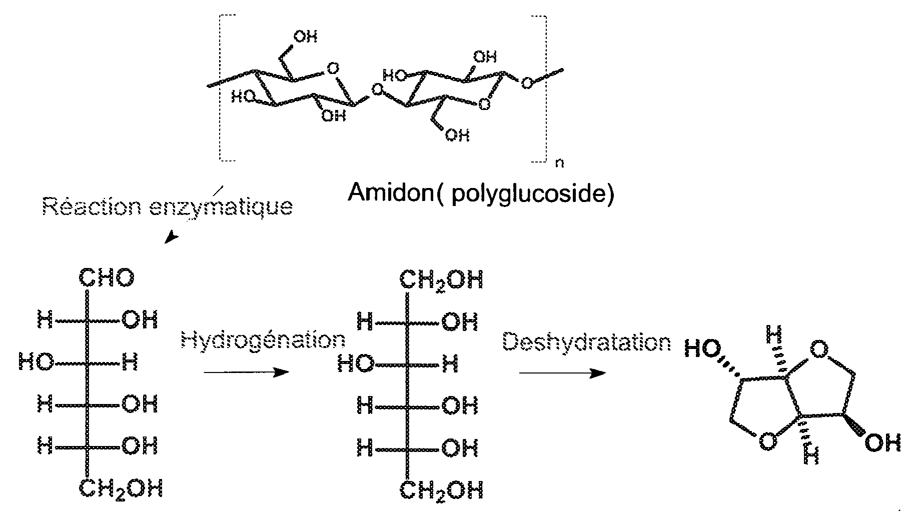

Les isomères optiques isomannide et isoidide sont obtenus de la même manière respectivement à partir du mannitol et de l'iditol. Pour plus de détails sur cette chimie, on peut se référer, entre autres, à l'encyclopédie KIRK OTHMER, 4^{ème} édition, Volume 23, pages 93 à 119.

A l'heure actuelle, dans le but de s'affranchir des dérivés du pétrole dans le contexte d'une « chimie verte », l'industrie des produits chimiques de performance est à la recherche de nouveaux composés ou monomères d'origine naturelle, telle que végétale, et donc renouvelables, biodégradables, peu toxiques et respectueux de l'environnement. Par ailleurs, ces nouveaux composés obtenus à partir de telles matières premières doivent, de préférence, pouvoir être obtenus au moyen de procédés propres et économes en énergie.

Compte tenu de ces exigences, la Société déposante a envisagé la synthèse de composés difonctionnels portant des groupements amines notamment, à partir des synthons naturels accessibles industriellement que sont l'isosorbide, l'isomannide et l'isoidide, et dont la disponibilité va augmenter ces prochaines années avec le développement des bioraffineries.

Les travaux de la Société déposante l'ont alors amené à trouver un procédé permettant de transformer les synthons ci-dessus à fonction alcool pour obtenir des nouveaux composés à fonctions nitriles et amines par un procédé simple et facilement développable à l'échelle industrielle.

Ce procédé repose sur le principe de transformer, dans une première étape, les fonctions alcools en éthers de propionitrile par réaction de Michael avec l'acrylonitrile, puis, dans une deuxième étape, à convertir les fonctions nitriles en fonctions amines primaires par hydrogénation.

Ainsi, à partir de sucres hétérocycloaliphatiques bicycliques, d'origine végétale, donc renouvelable, et disponibles industriellement à bas coût, la présente invention offre une voie d'accès simplifiée - synthèse aisée en deux étapes seulement, compatible avec les équipements industriels classiques - à des molécules originales, en particulier de part leur coeur bicyclique et leur stabilité thermique :
- Leur coeur bicyclique peut jouer, d'une part, le rôle d'une tête polaire assez grosse et hydrophile, et, d'autre part, dans le cas où il serait utilisé comme monomère, il peut assurer une certaine rigidité dans les matériaux.
- De façon surprenante, il a été observé que la tenue thermique des nouveaux composés de l'invention est excellente (étant supérieure à 296°C), ce qui est loin d'être le cas pour des produits sur base végétale.

A la connaissance de la Société déposante, les composés selon la présente invention sont nouveaux, n'étant jamais cités dans la littérature, à l'exception du 2,5-bis-O-(3-aminopropyl)-1,4:3,6-dianhydro-D-glucitol, lequel est certes cité par son N° CAS 6338-35-8, mais qui n'est décrit dans aucun document pas plus que son mode d'obtention.

Il existe une diamine dont les fonctions amines sont directement portées par le motif isosorbide, à savoir le 2,5-diamino-1,4:3,6-dianhydro-2,5-dideoxy-sorbitol ou 2,5-diamino-1,4:3,6-dianhydro-2,5-dideoxy-D-glucitol (CAS 143396-56-9). Cependant, comme on peut le voir dans les documents s'y rattachant, la synthèse est moins directe, à savoir en trois étapes et beaucoup plus compliquée car les rendements vont de 28 à 56% :
- Synthesis (1994), 317-321
- demande internationale PCT WO 9212978
- JACS (1956), 78, 3177-3182
- JCS (1950), 371-374
- Nature (1949), 164, 573-574.

On peut mentionner également l'article Bioorganic & Medecinal Chemistry Letters (2006), 16 (3), 714-717, qui traite de modélisation moléculaire de nouveaux ligands bis cationiques avec le site lipid A d'un lipopolysaccharide. Ce sont des études théoriques qui visent à obtenir des molécules ayant une longueur de 14 Angström en adéquation avec le site récepteur. On ne trouve aucune description de la molécule ou de son procédé de synthèse dans cet article.

Les nouvelles diamines de la présente invention trouvent une application comme tensio-actifs.

En effet, dans le domaine des tensio-actifs, les applications sont demandeuses de produits biodégradables et peu toxiques ayant, comme matières premières, des composés d'origine végétale et donc renouvelable. L'un des moyens de répondre à ce problème est d'utiliser une chimie de condensation entre, d'une part, une chaîne grasse lipophile - provenant des acides gras - répondant à ces critères et un synthon aminé hydrophile reliés entre eux par une fonction chimique clivable, telle que la fonction amide. Généralement les polyamines utilisées : diéthylènetriamine (DETA), triéthylènetétramine (TETA) etc ..., sont d'origine pétrolière et ont un impact sur l'environnement. La présente invention permet donc d'obtenir facilement une diamine sur base de matière première végétale répondant aux critères de biodégradabilité et de faible toxicité.

Les nouvelles diamines de l'invention ont aussi une application, dans le domaine des matériaux, en tant que monomères dans des réactions de polymérisation par condensation, par exemple pour la fabrication des polyamides, et aussi en tant qu'agents réticulants. Leur très bonne stabilité thermique et leur origine végétale constituent des critères de choix dans de telles applications.

La présente invention a donc d'abord pour objet des composés de formule (I) :

R-(CH₂)₂O-A-O-(CH₂)₂-R (I)

dans laquelle :
- A représente un radical divalent choisi parmi : 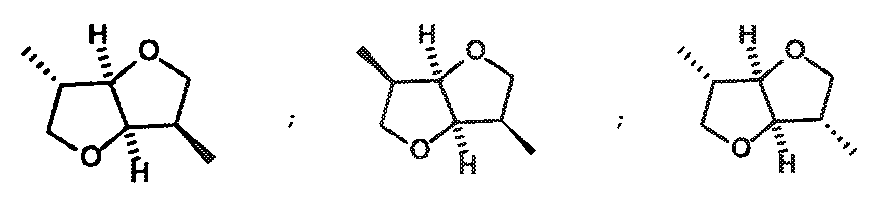 les deux liaisons libres dans chacune des trois formules ci-dessus constituant les points de rattachement du groupement A aux atomes d'oxygène dans la formule (I).
   et
- R représente -CN ou -CH₂NH₂.

On mentionne en particulier les composés de la présente invention représentés par les formules :

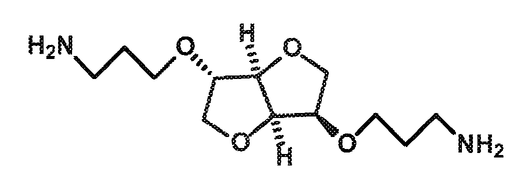

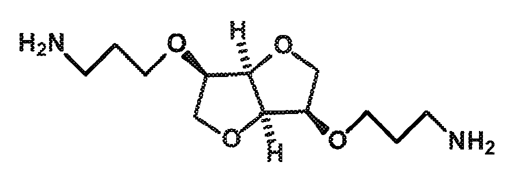

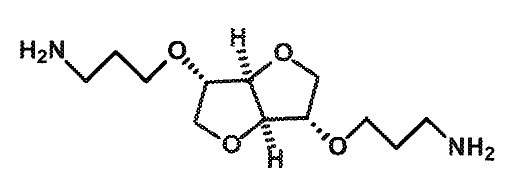

La présente invention a également pour objet un procédé de fabrication d'un composé de formule (I) tel que défini ci-dessus, caractérisé par le fait que l'on amène à réagir, par réaction de Michael, de l'acrylonitrile sur un composé de formule (II) :

HO-A-OH (II)

dans laquelle A est tel que défini à la revendication 1 pour obtenir un composé de formule (I) dans laquelle R représente -CN, et que l'on réalise l'hydrogénation de ce dernier pour obtenir le composé correspondant de formule (I) dans laquelle R représente -CH₂NH₂.

### Première étape : Réaction de Michael

On fait réagir l'acrylonitrile sur le composé de formule (II) notamment avec un rapport molaire acrylonitrile/(composé (II) x 2) de 1 à 2, de préférence de 1 à 1,5.

On conduit cette étape généralement à une température de 20°C à 100°C, de préférence de 40°C à 80°C.

Egalement, on conduit avantageusement cette étape en présence d'au moins un catalyseur basique ou non, utilisé notamment à raison de 0,05% à 5% en poids, de préférence de 0,1 à 3% en poids, par rapport au composé de formule (II).

On peut choisir le ou les catalyseurs basiques parmi :
- les hydroxydes de métaux alcalins, tels que les hydroxydes de Li, Na, K, Rb ou Cs ;
- les hydroxydes de métaux alcalino-terreux, tels que les hydroxydes de Mg, Ca, Sr ou Ba ;
- les carbonates de Li, Na, K, Rb ou Cs ;
- les alcoolates alcalins ou alcalino-terreux, tels que le méthylate de sodium, l'éthylate de sodium et le tertiobutylate de potassium ; et
- les catalyseurs hétérogènes basiques, tels que les résines basiques, les zéolithes, l'hydrotalcite et l'oxyde de magnésium ;

Parmi les autres catalyseurs non basiques (incluant ceux à caractère basique moins marqué), on peut citer le fluorure de K et le fluorure de Cs, purs ou supportés par exemple sur alumine.

Conformément à un premier mode de réalisation, on met en oeuvre le composé de formule (II) seul à l'état fondu.

Conformément à un second mode de réalisation, on met en oeuvre le composé de formule (II) en solution dans un solvant tel que le tertiobutanol dans le cas d'une réaction de Michael à basse température, les hydrocarbures aromatiques, tels que le toluène, et les solvants aprotiques polaires, tels que l'acétonitrile.

Enfin, on conduit généralement cette réaction de Michael à la pression atmosphérique, mais il n'y a pas d'inconvénient à travailler sous légère pression à cause du point d'ébullition de l'acrylonitrile qui est de 77°C.

On peut décrire de façon plus détaillée cette première étape, comme suit :
L'isosorbide ou son isomère isomannide ou isoidide peut être utilisé seul à l'état fondu (Pf : 60-63°C) dans le cas de l'isosorbide ou en solution dans un solvant, tel que le tertiobutanol pour les basses températures, c'est-à-dire à une température inférieure au point de fusion de la matière première, les hydrocarbures aromatiques (par exemple le toluène), les solvants aprotiques polaires (par exemple l'acétonitrile). On introduit un catalyseur basique qui est généralement un hydroxyde alcalin ou alcalino-terreux, tel que ceux indiqués plus haut, mais on peut aussi utiliser des alcoolates alcalins ou alcalinoterreux ainsi que des catalyseurs hétérogènes basiques, les carbonates alcalins ou les fluorures de potassium et de césium dont des exemples sont cités plus haut. Le mélange est chauffé entre 40°C et 80°C, puis on introduit l'acrylonitrile. La réaction est poursuivie jusqu'à conversion des fonctions alcools en éthers. Le produit de réaction peut être utilisé brut, mais on peut aussi le purifier par distillation sous vide poussé. On peut aussi neutraliser le catalyseur par un acide.

### Deuxième étape : Hydrogénation

On conduit avantageusement l'hydrogénation en présence d'ammoniac, avec un rapport molaire NH₃/CN généralement de 0,2 à 2,5, de préférence de 0,5 à 1,5.

On conduit cette hydrogénation dans les conditions avantageuses suivantes :
- à une température généralement de 40°C à 180°C, de préférence de 50°C à 130°C ;
- dans un réacteur sous pression à une pression totale de 5x10⁵ Pa à 1,50x10⁷ Pa (5 bars à 150 bars), de préférence de 2x10⁶ Pa à 8x10⁶ Pa (20 bars à 80 bars) ;
- en présence d'au moins un catalyseur d'hydrogénation, à raison notamment de 0,1 à 20% en poids, de préférence de 0,5% à 10% en poids par rapport au composé de formule (I) dans lequel R représente -CN, le ou les catalyseurs d'hydrogénation étant avantageusement choisis parmi le nickel de Raney, le cobalt de Raney, le palladium et le rhodium, ces deux derniers catalyseurs pouvant être supportés sur charbon ou alumine.

Conformément à un premier mode de réalisation, on conduit l'hydrogénation sans solvant.

Conformément à un second mode de réalisation, on conduit l'hydrogénation en milieu solvant, le ou les solvants étant compatibles avec la réaction d'hydrogénation et étant choisis notamment parmi l'eau et les alcools légers en C₁ à C₅, linéaires ou ramifiés.

On peut décrire de façon plus particulière et détaillée cette deuxième étape comme suit :
On utilise un réacteur sous pression. On peut opérer sans solvant ou en milieu solvant, des solvants utilisables à titre d'exemples étant cités plus haut. On charge dans le réacteur l'éther dinitrile et le catalyseur. Le catalyseur est choisi parmi les catalyseurs classiques d'hydrogénation des nitriles, tels que ceux cités plus haut. On préfère pour des raisons de coût le nickel de Raney et le cobalt de Raney. Le réacteur est refermé puis on introduit de l'ammoniac. Le milieu réactionnel est mis sous agitation et porté en température entre 50°C et 150°C. Puis on introduit l'hydrogène. La réaction démarre et est poursuivie jusqu'à conversion complète des fonctions nitriles en fonctions amines. La quantité d'ammoniac est judicieusement choisie de façon à minimiser la formation d'amine secondaire. En fin de réaction, on filtre le catalyseur qui peut être recyclé. On évapore le solvant s'il y a lieu. On purifie ou non la diamine par distillation sous vide poussé ou recristallisation de sa forme chlorhydrate.

On peut aussi opérer selon une variante de ce procédé qui consiste à charger dans le réacteur un solvant, le catalyseur, l'ammoniac, de l'hydrogène et introduire en continu l'éther dinitrile et de l'hydrogène pour maintenir la pression de la réaction. Le but est là aussi de favoriser la formation d'amines primaires au détriment d'amines secondaires.

La présente invention porte également sur l'utilisation d'un composé de formule (I) dans laquelle R représente -CH₂NH₂ comme tête polaire dans un tensio-actif, ou comme monomère (comonomère) d'une polymérisation par condensation, en particulier dans la fabrication de polyamides, ou encore comme agent réticulant, ainsi que sur l'utilisation d'un composé de formule (I) dans laquelle R représente -CN comme intermédiaire de synthèse de la préparation des composés de formule (I) dans laquelle R représente -CH₂NH₂.

Les Exemples suivants illustrent la présente invention sans toutefois en limiter la portée. Dans ces Exemples, les pourcentages sont en poids sauf indication contraire.

### Exemple 1 :

### Synthèse du 2,5-bis-O-(propionitrile)-1,4 :3,6-dianhydro-D-sorbitol :

Dans un réacteur en verre double enveloppe de 500 cm³, muni d'une agitation, d'une ampoule de coulée, d'un réfrigérant, on charge 100 g (0,68M) d'isosorbide et 0,5 g, soit 5000 ppm, de soude en perles. Le milieu réactionnel est porté à 70-75°C jusqu'à dissolution complète de la soude et fusion de l'isosorbide. Puis on additionne lentement 90,1 g (1,7M) soit 25% d'excès d'acrylonitrile par rapport aux fonctions alcool. En fin de réaction, on évapore l'excès d'acrylonitrile et on récupère le brut réactionnel. Le rendement en produit attendu est de 90%.

### Caractérisation analytique du produit :

### 2,5-bis-O-(propionitrile)-1,4 :3,6-dianhydro-D-sorbitol :

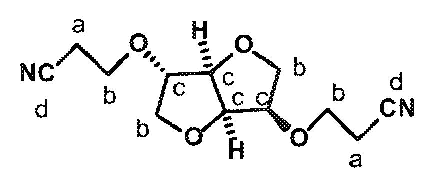

RMN ¹³C dans CDCl₃
δa = 18,31 ppm
δb = 63,95 ppm ; 64,51 ppm ; 70,02 ppm et 72,48 ppm
δc = 79,65 ppm ; 79,97 ppm ; 83,69 ppm et 85,35 ppm
δd = 117,48 ppm et 117,55 ppm.

### Exemple 2 :

### Synthese du 2,5-bis-O-(3-aminopropyl)-1,4 :3,6-dianhydro-D-sorbitol :

Dans un autoclave de 500 cm³, on introduit 200 g du brut réactionnel obtenu précédemment, avec 10 g de nickel de Raney humide. On referme l'autoclave. Puis on introduit 15 g d'ammoniac à température ambiante (soit un rapport molaire NH₃/CN de 0,55). Le milieu réactionnel est porté sous agitation à 130°C. La pression totale est amenée à 2,5x10⁶ Pa (25 bars) par introduction d'hydrogène.
La pression et la température sont maintenues à ces valeurs durant toute la réaction. Lorsque la réaction est terminée, on récupère le brut réactionnel par filtration afin de récupérer le catalyseur qui peut être recyclé. Le rendement est de 85%. La diamine peut être obtenue pure par distillation sous vide poussé. (Eb : 165-175°C sous 133,322 Pa (1 mm Hg)).

### Caractérisation analytique du produit :

### 2,5-bis-O-(3-aminopropyl)-1,4 :3,6-dianhydro-D-sorbitol :

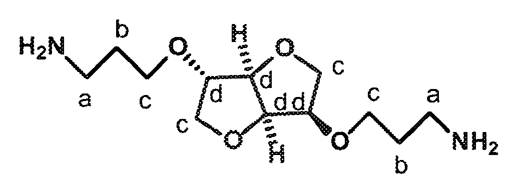

Confirmation de la masse par couplage CPV/SM
RMN ¹³C dans CD₃OD
δa = 40,42 ppm et 40,38 ppm
δb = 34,29 ppm et 34,11 ppm
δc = 69,16 ppm ; 69,97 ppm ; 71,69 ppm et 74,69 ppm
δd = 81,94 ppm ; 82,09 ppm ; 86,08 ppm et 87,85 ppm.

### Exemple 3 :

### Synthèse du 2,5-bis-O-(propionitrile)-1,4 :3,6-dianhydro-D-sorbitol :

Dans un réacteur en verre préalablement séché de 500 cm³, muni d'une agitation mécanique efficace, d'un chauffage, d'un réfrigérant, d'une ampoule de coulée, d'un système d'inertage à l'azote, on charge 46,2 g (316 mmoles) d'isosorbide avec 98,2 g de tertiobutanol et 1 g d'hydroxyde de lithium. Le milieu réactionnel est porté à 60°C, puis on coule 50,3 g (949 mmoles) d'acrylonitrile sur une durée de 1h30. La réaction est poursuivie sur une durée totale de 8h.

Le catalyseur est neutralisé par une solution acide puis on évapore sous pression réduite le tertiobutanol et l'acrylonitrile résiduel. On obtient ainsi 80,8 g de produit brut contenant 90% de dinitrile (dosage HPLC). La conversion de l'isosorbide est de 95% et le rendement est de 91 %.

### Caractérisation analytique du produit : cf Exemple 1.

### Exemple 4 :

### Synthèse du 2,5-bis-O-(3-aminopropyl)-1,4 :3,6-dianhydro-D-sorbitol :

Dans un autoclave de 300 cm³, on charge 100 g d'eau, 12 g de Nickel de Raney humide (50%), 2,6 g d'ammoniac. Le réacteur est pressurisé à l'hydrogène jusqu'à une pression totale de 6x10⁶ Pa (60 bars) pour une température de 60°C. On introduit en continu une solution de 34,5 g de brut réactionnel précédent dans 30 g d'eau. L'introduction se fait sur 3h15 et la pression et la température sont maintenues aux valeurs susmentionnées. En fin de réaction, le milieu est refroidi, le catalyseur, filtré, et le solvant, évaporé. On obtient ainsi 28,5 g de produit brut contenant 79% de diamine. Le rendement est de 70% en diamine.

Caractérisation analytique du produit : cf Exemple 2.

## Revendications

1. Composés de formule (I) :
R-(CH₂)₂-O-A-O-(CH₂)₂-R (I)
dans laquelle :
- A représente un radical divalent choisi parmi : 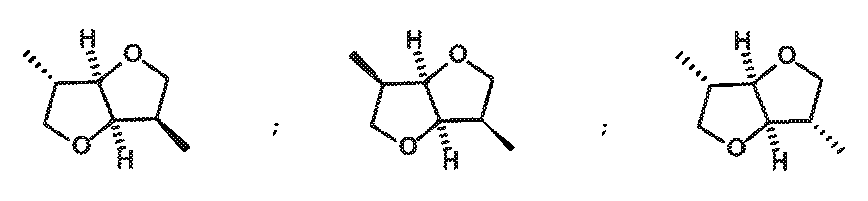 et
- R représente -CN ou -CH₂NH₂.

2. Composé selon la revendication 1, qui est représenté par la formule :

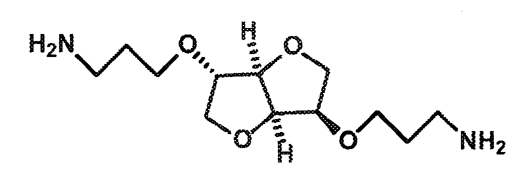

3. Composé selon la revendication 1, qui est représenté par la formule :

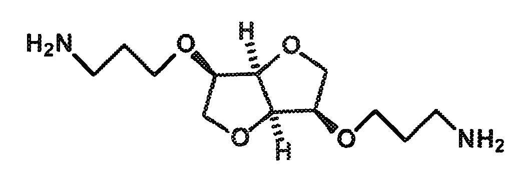

4. Composé selon la revendication 4, qui est représenté par la formule :

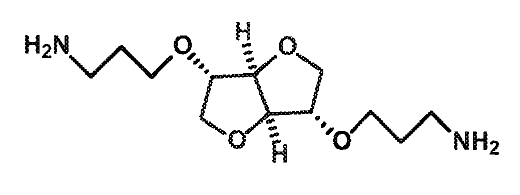

5. Procédé de fabrication d'un composé de formule (I) tel que défini à la revendication 1, **caractérisé par le fait que** l'on amène à réagir, par réaction de Michael, de l'acrylonitrile sur un composé de formule (II) :
HO-A-OH (II)
dans laquelle A est tel que défini à la revendication 1 pour obtenir un composé de formule (I) dans laquelle R représente -CN, et que l'on réalise l'hydrogénation de ce dernier pour obtenir le composé correspondant de formule (I) dans laquelle R représente -CH₂NH₂.

6. Procédé selon la revendication 5, **caractérisé par le fait que** l'on fait réagir l'acrylonitrile sur le composé de formule (II) avec un rapport molaire acrylonitrile/(composé (II) x 2) de 1 à 2, de préférence de 1 à 1,5.

7. Procédé selon l'une des revendications 5 et 6, **caractérisé par le fait que** l'on fait réagir l'acrylonitrile sur le composé de formule (II) à une température de 20°C à 100°C, de préférence de 40°C à 80°C.

8. Procédé selon l'une des revendications 5 à 7, **caractérisé par le fait que** l'on fait réagir l'acrylonitrile sur le composé de formule (II) en présence d'au moins un catalyseur basique ou non basique, utilisé notamment à raison de 0,05% à 5% en poids, de préférence de 0,1 à 3% en poids, par rapport au composé de formule (II).

9. Procédé selon la revendication 8, **caractérisé par le fait que** l'on choisit le ou les catalyseurs basiques parmi :
- les hydroxydes de métaux alcalins, tels que les hydroxydes de Li, Na, K, Rb ou Cs ;
- les hydroxydes de métaux alcalino-terreux, tels que les hydroxydes de Mg, Ca, Sr ou Ba ;
- les carbonates de Li, Na, K, Rb ou Cs ;
- les alcoolates alcalins ou alcalino-terreux, tels que le méthylate de sodium, l'éthylate de sodium et le tertiobutylate de potassium ; et
- les catalyseurs hétérogènes basiques, tels que les résines basiques, les zéolithes, l'hydrotalcite et l'oxyde de magnésium
et le ou les catalyseurs non basiques parmi le fluorure de K et le fluorure de Cs, purs ou supportés par exemple sur alumine.

10. Procédé selon l'une des revendications 5 à 9, **caractérisé par le fait que** l'on met en oeuvre le composé de formule (II) seul à l'état fondu.

11. Procédé selon l'une des revendications 5 à 9, **caractérisé par le fait que** l'on met en oeuvre le composé de formule (II) en solution dans un solvant tel que le tertiobutanol dans le cas d'une réaction de Michael à basse température, les hydrocarbures aromatiques, tels que le toluène, et les solvants aprotiques polaires, tels que l'acétonitrile.

12. Procédé selon l'une des revendications 5 à 11, **caractérisé par le fait que** l'on conduit la réaction de Michael à la pression atmosphérique ou sous légère pression.

13. Procédé selon l'une des revendications 5 à 12, **caractérisé par le fait que** l'on conduit l'hydrogénation en présence d'ammoniac, avec un rapport molaire NH₃/CN de 0,2 à 2,5, de préférence de 0,5 à 1,5.

14. Procédé selon l'une des revendications 5 à 13, **caractérisé par le fait que** l'on conduit l'hydrogénation à une température de 40°C à 180°C, de préférence de 50°C à 130°C.

15. Procédé selon l'une des revendications 5 à 14, **caractérisé par le fait que** l'on conduit l'hydrogénation dans un réacteur sous pression à une pression totale de 5x10⁵ Pa à 1,5x10⁷ Pa (5 bars à 150 bars), de préférence de 2x10⁶ Pa à 8x10⁶ Pa (20 bars à 80 bars).

16. Procédé selon l'une des revendications 5 à 15, **caractérisé par le fait que** l'on conduit la réaction en présence d'au moins un catalyseur d'hydrogénation, à raison notamment de 0,1 à 20% en poids, de préférence de 0,5% à 10 en poids, par rapport au composé de formule (I) dans lequel R représente -CN.

17. Procédé selon la revendication 16, **caractérisé par le fait que** l'on choisit le ou les catalyseurs d'hydrogénation parmi le nickel de Raney, le cobalt de Raney, le palladium et le rhodium, ces deux derniers catalyseurs pouvant être supportés sur charbon ou alumine.

18. Procédé selon l'une des revendications 5 à 17, **caractérisé par le fait que** l'on conduit l'hydrogénation sans solvant.

19. Procédé selon l'une des revendications 5 à 17, **caractérisé par le fait que** l'on conduit l'hydrogénation en milieu solvant, le ou les solvants étant compatibles avec la réaction d'hydrogénation et étant choisis notamment parmi l'eau et les alcools légers en C₁ à C₅, linéaires ou ramifiés.

20. Utilisation d'un composé de formule (I) dans laquelle R représente -CH₂NH₂ comme tête polaire dans un tensio-actif, ou comme monomère d'une polymérisation par condensation, en particulier dans la fabrication de polyamides, ou encore comme agent réticulant.

21. Utilisation d'un composé de formule (I) dans laquelle R représente -CN comme intermédiaire de synthèse de la préparation des composés de formule (I) dans laquelle R représente -CH₂NH₂.

## Claims

1. A compound of formula (I):
R-(CH₂)₂-O-A-O-(CH₂)₂-R (I)
in which:
- A represents a divalent radical chosen from: 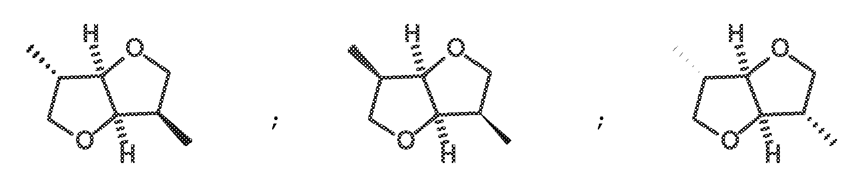 and
- R represents -CN or -CH₂NH₂.

2. The compound as claimed in claim 1, which is represented by the formula:

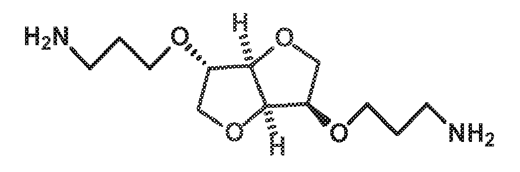

3. The compound as claimed in claim 1, which is represented by the formula:

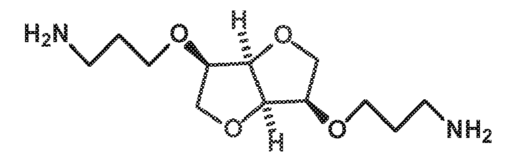

4. The compound as claimed in claim 1, which is represented by the formula:

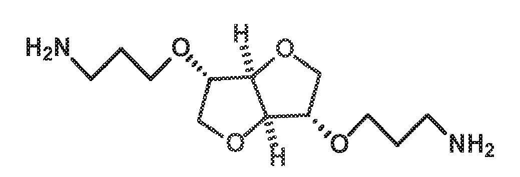

5. A process for manufacturing a compound of formula (I) as defined in claim 1, **characterized by** the fact that acrylonitrile is reacted, via Michael reaction, with a compound of formula (II):
HO-A-OH (II)
in which A is as defined in claim 1, in order to obtain a compound of formula (I) in which R represents -CN, and that the hydrogenation of the latter is carried out in order to obtain the corresponding compound of formula (I) in which R represents -CH₂NH₂.

6. The process as claimed in claim 5, **characterized by** the fact that acrylonitrile is reacted with the compound of formula (II) with an acrylonitrile/(compound (II) x 2) molar ratio of 1 to 2, preferably of 1 to 1.5.

7. The process as claimed in either of claims 5 and 6, **characterized by** the fact that the acrylonitrile is reacted with the compound of formula (II) at a temperature of 20°C to 100°C, preferably from 40°C to 80°C.

8. The process as claimed in one of claims 5 to 7, **characterized by** the fact that the acrylonitrile is reacted with the compound of formula (II) in the presence of at least one basic or non-basic catalyst, used in particular in an amount of 0.05 to 5% by weight, preferably 0.1 to 3% by weight, relative to the compound of formula (II).

9. The process as claimed in claim 8, **characterized by** the fact that the basic catalyst(s) is(are) chosen from:
- alkali metal hydroxides, such as Li, Na, K, Rb or Cs hydroxide;
- alkaline-earth metal hydroxides, such as Mg, Ca, Sr or Ba hydroxide;
- Li, Na, K, Rb or Cs carbonates;
- alkali or alkaline-earth metal alcoholates, such as sodium methylate, sodium ethylate and potassium *tert*-butylate; and
- basic heterogeneous catalysts, such as basic resins, zeolites, hydrotalcite and magnesium oxide,
and the non-basic catalyst(s) is(are) chosen from K fluoride and Cs fluoride, pure or supported, for example, on alumina.

10. The process as claimed in one of claims 5 to 9, **characterized by** the fact that use is made of the compound of formula (II) alone in the molten state.

11. The process as claimed in one of claims 5 to 9, **characterized by** the fact that use is made of the compound of formula (II) in solution in a solvent such as *tert*-butanol in the case of a low-temperature Michael reaction, aromatic hydrocarbons, such as toluene, and polar aprotic solvents, such as acetonitrile.

12. The process as claimed in one of claims 5 to 11, **characterized by** the fact that the Michael reaction is carried out at atmospheric pressure or under a slight pressure.

13. The process as claimed in one of claims 5 to 12, **characterized by** the fact that the hydrogenation is carried out in the presence of ammonia, with an NH₃/CN molar ratio of 0.2 to 2.5, preferably 0.5 to 1.5.

14. The process as claimed in one of claims 5 to 13, **characterized by** the fact that the hydrogenation is carried out at a temperature of 40°C to 180°C, preferably 50°C to 130°C.

15. The process as claimed in one of claims 5 to 14, **characterized by** the fact that the hydrogenation is carried out in a pressurized reactor at a total pressure of 5 × 10⁵ Pa to 1.5 × 10⁷ Pa (5 bar to 150 bar), preferably 2 × 10⁶ Pa to 8 × 10⁶ Pa (20 bar to 80 bar).

16. The process as claimed in one of claims 5 to 15, **characterized by** the fact that the reaction is carried out in the presence of at least one hydrogenation catalyst, in an amount, especially, of 0.1 to 20% by weight, preferably 0.5% to 10% by weight, relative to the compound of formula (I) in which R represents -CN.

17. The process as claimed in claim 16, **characterized by** the fact that the hydrogenation catalyst(s) is(are) chosen from Raney nickel, Raney cobalt, palladium and rhodium, the latter two catalysts possibly being supported on charcoal or alumina.

18. The process as claimed in one of claims 5 to 17, **characterized by** the fact that the hydrogenation is carried out without solvent.

19. The process as claimed in one of claims 5 to 17, **characterized by** the fact that the hydrogenation is carried out in a solvent medium, the solvent(s) being compatible with the hydrogenation reaction and being chosen, in particular, from water and linear or branched C₁ to C₅ light alcohols.

20. The use of a compound of formula (I) in which R represents -CH₂NH₂ as a polar head in a surfactant, or as a monomer for a condensation polymerization, in particular in the manufacture of polyamides, or else as a crosslinking agent.

21. The use of a compound of formula (I) in which R represents -CN as a synthesis intermediate in the preparation of compounds of formula (I) in which R represents -CH₂NH₂.

## Patentansprüche

1. Verbindungen der Formel (I):
R-(CH₂)₂-O-A-O-(CH₂)₂-R (I)
in der Formel:
- A bedeutet eine zweiwertige Gruppe, die ausgewählt ist unter: 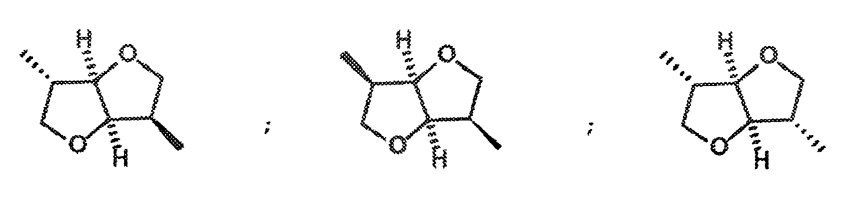 und
- R bedeutet -CN oder -CH₂NH₂.

2. Verbindung nach Anspruch 1, die durch die folgende Formel dargestellt wird:

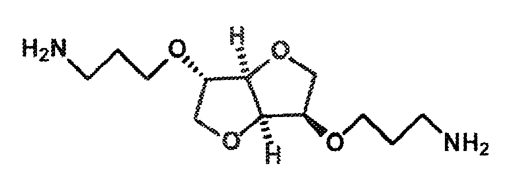

3. Verbindung nach Anspruch 1, die durch die folgende Formel dargestellt wird:

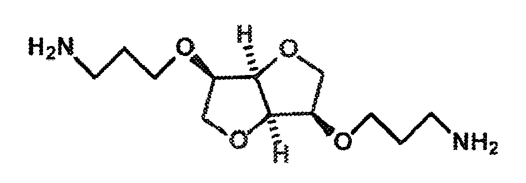

4. Verbindung nach Anspruch 1, die durch die folgende Formel dargestellt wird:

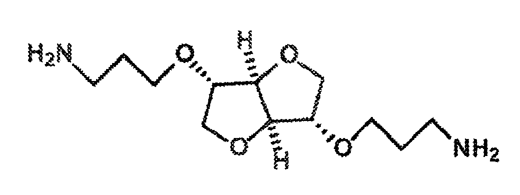

5. Verfahren zur Herstellung einer Verbindung der Formel (I), wie sie in Anspruch 1 definiert ist, **dadurch gekennzeichnet, dass** Acrylnitril gemäß einer Michael-Reaktion mit einer Verbindung der Formel (II) umgesetzt wird:
HO-A-OH (II)
wobei A die in Anspruch 1 angegebenen Bedeutungen aufweist, um eine Verbindung der Formel (I) zu bilden, bei der R -CN bedeutet, und **dadurch**, dass diese hydriert wird, um die entsprechende Verbindung der Formel (I) herzustellen, bei der R -CH₂NH₂ bedeutet.

6. Verfahren nach Anspruch 5, **dadurch gekennzeichnet, dass** das Acrylnitril mit der Verbindung (II) in einem Molverhältnis Acrylnitril/ (Verbindung (II) x 2) im Bereich von 1 bis 2 und vorzugsweise 1 bis 1,5 umgesetzt wird.

7. Verfahren nach einem der Ansprüche 5 oder 6, **dadurch gekennzeichnet, dass** das Acrylnitril mit der Verbindung (II) bei einer Temperatur von 20 bis 100 °C und vorzugsweise 40 bis 80 °C umgesetzt wird.

8. Verfahren nach einem der Ansprüche 5 bis 7, **dadurch gekennzeichnet, dass** das Acrylnitril mit der Verbindung (II) in Gegenwart mindestens eines basischen oder nicht basischen Katalysators umgesetzt wird, der insbesondere in einer Menge von 0,05 bis 5 Gew.-% und vorzugsweise 0,1 bis 3 Gew.-%, bezogen auf die Verbindung der Formel (II), verwendet wird.

9. Verfahren nach Anspruch 8, **dadurch gekennzeichnet, dass** der oder die basischen Katalysatoren ausgewählt sind unter:
- Hydroxiden von Alkalimetallen, wie Hydroxiden von Li, Na, K, Rb oder Cs;
- Hydroxiden von Erdalkalimetallen, wie Hydroxiden von Mg, Ca, Sr oder Ba;
- Carbonaten von Li, Na, K, Rb oder Cs;
- Alkali- oder Erdalkalialkoholaten, wie Natriummethanolat, Natriumethanolat und Kalium-tert-butanolat; und
- heterogenen basischen Katalysatoren, wie basischen Harzen, Zeolithen, Hydrotalcit und Magnesiumoxid;
und der oder die nicht basischen Katalysatoren unter dem Fluorid von K und dem Fluorid von Cs ausgewählt sind, wobei sie in reiner Form oder auf einem Träger wie beispielsweise Aluminiumoxid vorliegen.

10. Verfahren nach einem der Ansprüche 5 bis 9, **dadurch gekennzeichnet, dass** die Verbindung der Formel (II) in geschmolzenem Zustand alleine verwendet wird.

11. Verfahren nach einem der Ansprüche 5 bis 9, **dadurch gekennzeichnet, dass** die Verbindung der Formel (II) in einem Lösemittel gelöst verwendet wird, wie beispielsweise tert-Butanol im Falle einer Michael-Reaktion bei niedriger Temperatur, aromatischen Kohlenwasserstoffen, wie Toluol, und aprotischen polaren Lösemitteln, wie Acetonitril.

12. Verfahren nach einem der Ansprüche 5 bis 11, **dadurch gekennzeichnet, dass** die Michael-Reaktion bei Atmosphärendruck oder bei einem leichten Druck durchgeführt wird.

13. Verfahren nach einem der Ansprüche 5 bis 12, **dadurch gekennzeichnet, dass** die Hydrierung in Gegenwart von Ammoniak in einem Molverhältnis NH₃/CN von 0,2 bis 2,5 und vorzugsweise 0,5 bis 1,5 erfolgt.

14. Verfahren nach einem der Ansprüche 5 bis 13, **dadurch gekennzeichnet, dass** die Hydrierung bei einer Temperatur von 40 bis 180 °C und vorzugsweise 50 bis 130 °C erfolgt.

15. Verfahren nach einem der Ansprüche 5 bis 14, **dadurch gekennzeichnet, dass** die Hydrierung unter Druck in einem Rektor bei einem Gesamtdruck von 5x10⁵ bis 1,5x10⁷ Pa (5 bis 150 bar) und vorzugsweise 2x10⁶ bis 8x10⁶ Pa (20 bis 80 bar) durchgeführt wird.

16. Verfahren nach einem der Ansprüche 5 bis 15, **dadurch gekennzeichnet, dass** die Reaktion in Gegenwart mindestens eines Hydrierungskatalysators insbesondere in einem Mengenanteil von 0,1 bis 20 Gew.-% und vorzugsweise 0,5 bis 10 Gew.-%, bezogen auf die Verbindung der Formel (I), worin R -CN bedeutet, durchgeführt wird.

17. Verfahren nach Anspruch 16, **dadurch gekennzeichnet, dass** der oder die Hydrierungskatalysatoren unter Raney-Nickel, Raney-Cobalt, Palladium und Rhodium ausgewählt sind, wobei die beiden zuletzt genannten Katalysatoren auf Kohlenstoff oder Aluminiumoxid als Träger vorliegen können.

18. Verfahren nach einem der Ansprüche 5 bis 17, **dadurch gekennzeichnet, dass** die Hydrierung ohne Lösemittel erfolgt.

19. Verfahren nach einem der Ansprüche 5 bis 17, **dadurch gekennzeichnet, dass** die Hydrierung in einem Lösemittelmedium durchgeführt wird, wobei das oder die Lösemittel mit der Hydrierungsreaktion kompatibel sind und insbesondere unter Wasser und den geradkettigen oder verzweigten niedrigsiedenden Alkoholen mit 1 bis 5 Kohlenstoffatomen ausgewählt sind.

20. Verwendung einer Verbindung der Formel (I), worin R -CH₂NH₂ bedeutet, als polaren Kopf in einem grenzflächenaktiven Stoff oder als Monomer in einer Kondensationspolymerisation, insbesondere zur Herstellung von Polyamiden, oder als Vernetzungsmittel.

21. Verwendung einer Verbindung der Formel (I), worin R -CN bedeutet, als Synthesezwischenprodukt bei der Herstellung von Verbindungen der Formel (I), worin R -CH₂NH₂ bedeutet.
